# FASCICULE DE BREVET EUROPEEN

(11) **EP 1 120 099 B1**
(45) Date de publication et mention de la délivrance du brevet: **12.04.2006**
(21) Numéro de dépôt: 00390011.5
(22) Date de dépôt: 07.07.2000
(51) Int. Cl.: A61J 3/00, A61M 1/16

(54) **Cartouche pour la préparation d'une solution à usage médical**
Behälter zur Herstellung einer medizinischen Lösung
Cartridge for preparation of a medical solution

(30) Priorité: 24.01.2000 CA 2297271
(43) Date de publication de la demande: 01.08.2001
(73) Titulaire: Laboratoire Soludia, 31450 Montgiscard (FR)
(72) Inventeur: Lascombes, Jean-Jacques, 31000 Toulouse (FR)
(74) Mandataire: Morelle, Guy Georges Alain

(56) Documents cités:
- GB-A- 2 309 681
- US-A- 2 731 013
- US-A- 4 949 873
- US-A- 5 318 750

## Description

La présente invention concerne les cartouches permettant de réaliser une solution à usage médical par dissolution d'une pluralité de substances en poudre dans au moins un liquide, et plus particulièrement les cartouches permettant de réaliser une solution pour dialyse.

On sait que, dans de nombreux domaines, notamment dans le domaine médical, il est nécessaire de préparer des solutions suivant des dosages bien déterminés pour effectuer des traitements particuliers. Tel est par exemple le cas des solutions pour des séances d'hémodialyse.

De nombreux procédés et dispositifs ont déjà été mis au point pour réaliser de telles solutions à usage médical, par exemple celui qui est décrit dans le US-A-5,318,750, au nom du demandeur.

La préparation d'une solution à usage médical par dissolution dans un fluide porteur de plusieurs substances sous forme de poudre, décrit dans ce document, s'effectue au moyen d'un dispositif dans lequel ces substances sont contenues dans des cellules et qui comprend une première conduite communiquant avec une entrée des cellules pour introduire le fluide porteur dans les cellules afin de produire des solutés dans les cellules, une seconde conduite communiquant avec une sortie des cellules pour amener les solutés produits dans les cellules vers un point de mélange situé en amont d'un lieu d'utilisation, un moyen de mesure pour mesurer la concentration des solutés et un moyen de régulation de débit pour modifier la concentration des solutés en réponse aux informations fournies par au moins le moyen de mesure, l'ensemble des substances nécessaires à la préparation de la solution étant dissout dans les cellules et chaque cellule contenant au moins l'une de ces substances sous forme de poudre.

II faut savoir aussi que ces cellules sont destinées à être utilisées dans un milieu hospitalier ou analogue, dans lequel il est absolument nécessaire qu'il y ait le moins de manipulations possibles pour effectuer la préparation nécessaire à chaque traitement. Il faut aussi que les quelques manipulations qu'il est obligatoirement nécessaire d'effectuer soient les plus simples possibles et rapides, pour faciliter le travail du personnel soignant et réduire au maximum le temps d'intervention sur le malade pour son traitement.

Aussi, la présente invention a-t-elle pour but de réaliser une cartouche pour la préparation d'une solution à usage médical par dissolution d'une pluralité de substances en poudre dans au moins un liquide, et plus particulièrement les cartouches permettant de réaliser une solution pour dialyse, qui soit facilement utilisable par les praticiens qui doivent effectuer des interventions sur des patients au moyen des produits contenus dans ces cartouches.

Un autre but de la présente invention est de réaliser une telle cartouche à usage médical qui soit apte à être livrée sous forme dite sèche et stérile, en étant en outre prête immédiatement à l'emploi par les praticiens.

Un autre but de la présente invention est de réaliser une telle cartouche à usage médical qui soit commode à manipuler par les praticiens sans qu'ils aient à prendre des précautions de manipulations particulières autres que celles qui sont nécessaire à la pratique de leur intervention.

Un autre but de la présente invention est de réaliser une telle cartouche à usage médical qui puisse être facilement stockée tout en étant prête immédiatement à l'emploi sans opération particulière longue et délicate.

Un autre but de la présente invention est de réaliser une telle cartouche à usage médical qui, tout à la fois, protège particulièrement bien les produits en poudre en attente de leur utilisation et soit facilement réutilisable après avoir été remplie à nouveau.

Un autre but de la présente invention est de réaliser une telle cartouche à usage médical qui soit facilement stérilisable et facilement encapsulable après ou avant stérilisation.

Plus précisément, la présente invention a pour objet une cartouche permettant de réaliser une solution à usage médical par dissolution d'une pluralité de substances en poudre dans au moins un liquide, et qui comporte :
un récipient définissant un volume principal, ledit récipient comportant une paroi latérale de forme sensiblement de révolution délimitant, à une de ses deux extrémités, une ouverture, une paroi de fond solidaire de l'autre extrémité de ladite paroi latérale, les parois latérale et de fond délimitant ledit volume principal, un noyau central de forme sensiblement de révolution, ledit noyau central étant solidaire, par une de ses deux extrémités, de ladite paroi de fond de façon que son axe de révolution soit sensiblement confondu avec celui de la paroi latérale, la hauteur dudit noyau central étant sensiblement égale à celle de ladite paroi latérale pour définir un volume annulaire dans ledit volume principal, au moins deux parois de séparation solidaires de façon étanche de la paroi de fond, de la surface intérieure de la paroi latérale et de la surface extérieure dudit noyau central pour délimiter deux volumes secondaires dans ledit volume annulaire, et
au moins deux raccords fluidiques montés en coopération avec la paroi de fond, ces deux raccords fluidiques permettant de relier l'extérieur dudit récipient avec respectivement les deux volumes secondaires.

La présente invention a aussi pour objet une cartouche permettant de réaliser une solution pour dialyse, et qui comporte :
un récipient définissant un volume principal, ledit récipient comportant une paroi latérale de forme sensiblement de révolution délimitant, à une de ses deux extrémités, une ouverture, une paroi de fond solidaire de l'autre extrémité de ladite paroi latérale, les parois latérale et de fond délimitant ledit volume principal, un noyau central de forme sensiblement de révolution, ledit noyau central étant solidaire, par une de ses deux extrémités, de ladite paroi de fond de façon que son axe de révolution soit sensiblement confondu avec celui de la paroi latérale, la hauteur dudit noyau central étant sensiblement égale à celle de ladite paroi latérale pour définir un volume annulaire dans ledit volume principal, quatre parois de séparation solidaires de façon étanche de la paroi de fond, de la surface intérieure de la paroi latérale et de la surface extérieure dudit noyau central pour délimiter quatre volumes secondaires dans ledit volume annulaire, et
au moins quatre raccords fluidiques montés en coopération avec la paroi de fond, ces quatre raccords fluidiques permettant de relier l'extérieur dudit récipient avec respectivement les quatre volumes secondaires.

D'autres caractéristiques et avantages de la présente invention apparaîtront au cours de la description suivante donnée en regard des dessins annexés à titre illustratif mais nullement limitatif, dans lesquels :

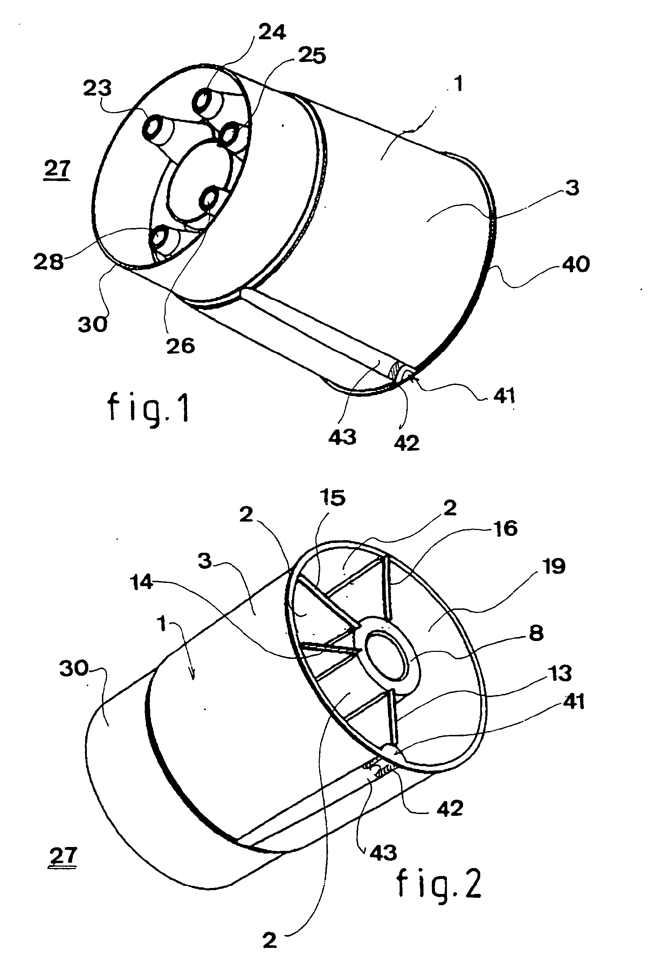

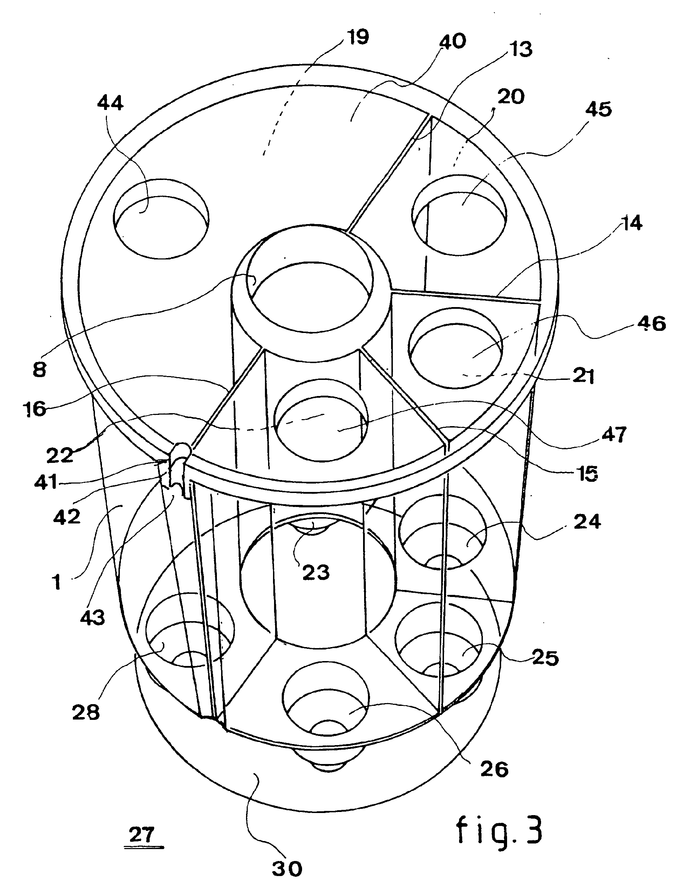

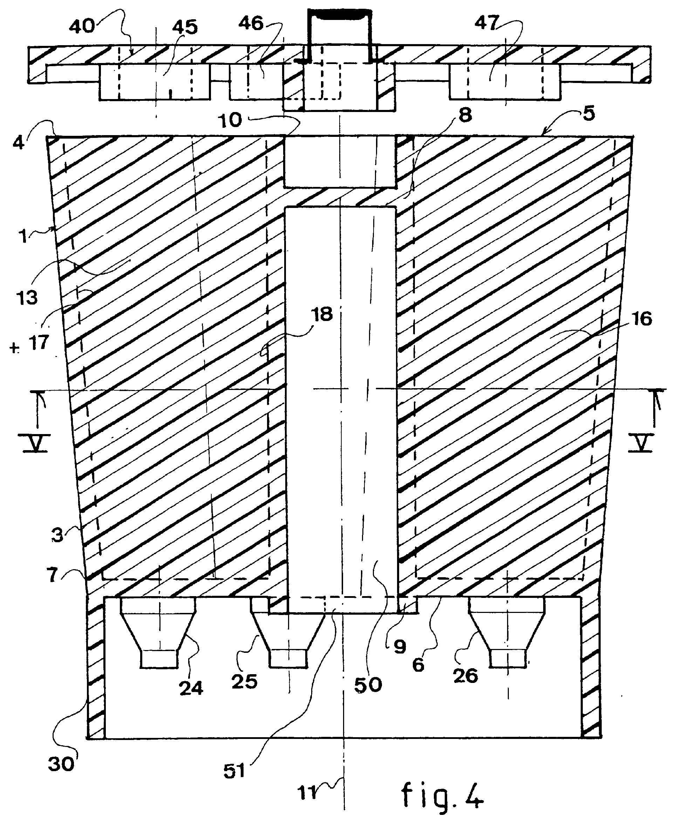

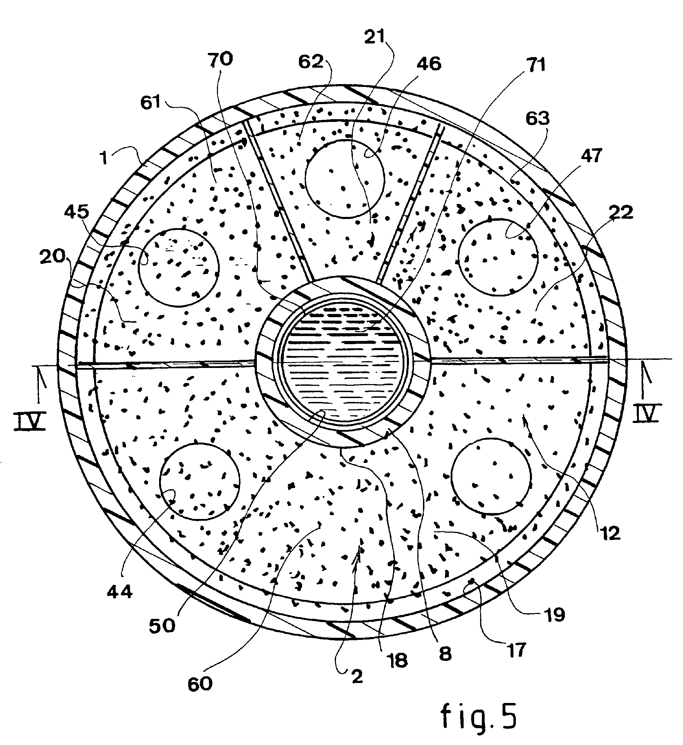

Les figures 1 à 5 représentent un mode de réalisation d'une cartouche selon l'invention, les figures 1 et 2 représentent ce mode dans deux vues en perspectives cavalières suivant deux angles de vue différents en précisant que la vue 2 ne comporte pas l'élément constitué par un couvercle, la vue selon la figure 3 étant une vue en transparence, et les figures 4 et 5 représentant ce mode de réalisation selon deux coupes suivant deux plans perpendiculaires, la figure 4 étant une coupe longitudinale référencée IV-IV sur la figure 5 et la figure 5 étant une coupe transversale référencée V-V sur la figure 4.

Les figures 1 à 5 ne représentent qu'un mode de réalisation d'une cartouche selon l'invention et dans le but de faciliter la compréhension de la présente description, les mêmes références y désignent dans ces figures les mêmes éléments, quelle que soit la figure sur laquelle elles apparaissent. De même, si des éléments ne sont pas spécifiquement référencés sur l'une des figures, leur référence peut être aisément retrouvée en se reportant à l'autre figure.

Le demandeur tient à préciser que les figures 1 et 2 ne représentent qu'un mode de réalisation de l'objet selon l'invention et qu'il peut exister d'autres modes de réalisation qui répondent à la définition de cette invention.

Il précise en outre que, lorsque, selon la définition de l'invention, l'objet de l'invention comporte "au moins un" élément ayant une fonction donnée, le mode de réalisation décrit peut comporter plusieurs de ces éléments.

Il précise aussi que, si le mode de réalisation de l'objet selon l'invention tel qu'illustré comporte plusieurs éléments de fonction identique et que si, dans la description, il n'est pas spécifié que l'objet selon cette invention doit obligatoirement comporter un nombre particulier de ces éléments, l'objet de l'invention pourra être défini comme comportant "au moins un" de ces éléments.

Ceci ayant été précisé, la présente invention concerne une cartouche permettant de réaliser une préparation d'une solution à usage médical par dissolution d'une pluralité de substances en poudre dans au moins un liquide.

Cette cartouche comporte, par référence à toutes les figures, un récipient 1 définissant un volume principal 2. Le récipient comporte une paroi latérale 3 de forme sensiblement de révolution délimitant, à une 4 de ses deux extrémités 4, et 7 une ouverture 5, une paroi de fond 6 solidaire de l'autre extrémité 7 de cette paroi latérale, les parois latérale et de fond délimitant le volume principal 2 défini ci-dessus, un noyau central 8 de forme sensiblement de révolution, ce noyau central étant solidaire, par une de ses deux extrémités 9, 10, de la paroi de fond 6 de façon que son axe de révolution 11 soit sensiblement confondu avec celui de la paroi latérale 3, la hauteur du noyau central étant sensiblement égale à celle de la paroi latérale pour définir un volume annulaire 12 entièrement contenu dans le volume principal 2, au moins deux parois de séparation 13, 14, 15, 16 solidaires de façon étanche de la paroi de fond 6, de la surface intérieure 17 de la paroi latérale 3 et de la surface extérieure 18 dudit noyau central 8 pour délimiter deux volumes secondaires 19-22 dans ce volume annulaire 12.

La cartouche comporte en outre au moins deux raccords fluidiques 23-26 montés en coopération avec la paroi de fond 6, ces deux raccords fluidiques permettant de relier l'extérieur 27 du récipient 1 avec respectivement les deux volumes secondaires 19-22. Ces raccords peuvent être de tous types, notamment de la forme comme ceux illustrés sur les figures, de façon à pouvoir connecter par exemple une conduite de fluide avec ces raccords, de façon à pouvoir alimenter les volumes secondaires avec ce fluide et obtenir comme il sera précisé ci-après, une dissolution de produit en poudre contenu dans ces volumes secondaires.

De façon préférentielle la cartouche comporte une couronne de protection 30 solidaire de la paroi de fond 6 et entourant les raccords fluidiques 23-26, la hauteur de la couronne prise à partie de ladite paroi de fond étant supérieure à la hauteur desdits raccords fluidiques prise à partir de cette même paroi de fond. De cette façon la cartouche peut reposer sur une surface de sol sans que les raccords viennent au contact de ce sol, et il est donc conféré à cette cartouche sur ce plan un protection qui est importante pour son utilisation ultérieure.

En outre, il est aussi avantageux que la cartouche comporte, comme plus particulièrement illustré suer les figures 1, 3 et 4, un couvercle 40 apte à recouvrir l'ouverture 5 du récipient 1.

De plus, il est aussi prévu qu'elle comporte des moyens 41 pour indexer angulairement la position du couvercle 40 sur l'ouverture 5 par rapport à la paroi latérale 3 pour que le couvercle soit dans une position déterminée par rapport au récipient. Ces moyens 41 peuvent être constitués de différentes façons mais avantageusement comme illustré plus particulièrement sur les figures 1, 2 et 3, par au moins une partie en saillie 42 réalisée sur l'un des deux éléments "couvercle" et "paroi latérale" en l'occurrence, sur le mode de réalisation illustré, sur le couvercle 40 et par une partie en creux 43 complémentaire de la partie en saillie, la partie en creux étant réalisée sur l'autre des deux éléments c'est-à-dire dans ce mode de réalisation sur la paroi latérale 3.

De cette façon, avec ces moyens d'indexation 41, il est donc possible que le couvercle 40 comporte au moins deux orifices d'alimentation 44-47, par exemple en poudre ou analogue, ces deux orifices étant alors, de ce fait, respectivement disposés en regard des deux volumes secondaires 19-22 pour permettre de les remplir ou de compléter leur remplissage si nécessaire.

Ces cartouches sont destinées comme mentionné auparavant à réaliser des solutions à usage médical par dissolution d'une pluralité de substances en poudre dans au moins un liquide, plus précisément de l'eau stérilisée. Mais il est parfois nécessaire d'utiliser un autre fluide spécifique. Aussi il est très intéressant que la cartouche puise livrer ce fluide spécifique et qu'il soit transporté avec celle-ci en même temps.

Aussi de façon très avantageuse, la cartouche comporte un logement 50 réalisé dans le noyau central 8 qui peut servir directement ou indirectement de réserve pour ce fluide spécifique et donc il est avantageux que ce logement comporte une ouverture 51 réalisée au niveau de la paroi de fond.

La cartouche décrite ci-dessus permet la réalisation de solutions à usage médical par dissolution d'une pluralité de substances en poudre dans au moins un liquide. Cependant elle trouve une application particulièrement avantageuse dans la réalisation de solutions pour dialyse.

Une cartouche pour cette application est plus particulièrement représentée sur les figures 1 à 5. Aussi pour cette application, elle comporte plus particulièrement quatre parois de séparation 13-16 solidaires de façon étanche de la paroi de fond 6, de la surface intérieure 17 de la paroi latérale 3 et de la surface extérieure 18 dudit noyau central 8 pour délimiter quatre volumes secondaires 19-22 dans le volume annulaire 12, et donc de ce fait au moins quatre raccords fluidiques 23-26 montés en coopération avec la paroi de fond 6, ces quatre raccords fluidiques permettant de relier l'extérieur 27 du récipient 1 avec respectivement les quatre volumes secondaires 19-22.

Dans cette réalisation définie ci-dessus, les premier 19, deuxième 20, troisième 21 et quatrième 22 volumes secondaires 19-22 représentent respectivement 50%, 20%. 10% et 20% du volume annulaire 12, en outre le premier volume secondaire 19 ayant un volume représentant 50% du volume annulaire contient du chlorure de sodium 60, les deuxième 20 et quatrième 22 volumes secondaires ayant chacun un volume égal à 20% du volume annulaire contiennent respectivement du chlorure de potassium 61 et du chlorure de calcium 62, et le troisième volume secondaire 21 ayant un volume égal à 10% du volume annulaire contient du chlorure de magnésium 63. Ces différents produits ont été uniquement évoqués schématiquement sur la figure 5 qui est une coupe transversale de la cartouche, en précisant que les autres figures 1 à 4, représentent la cartouche selon l'invention sans les produits en poudre, de façon à simplifier ces figures et faciliter leur compréhension.

De façon parfaitement adaptée pour la réalisation d'une séance de dialyse, les dimensions des premier, deuxième, troisième et quatrième volume secondaires 19-22 sont respectivement déterminées pour que la masse du chlorure de sodium contenu dans le premier volume secondaire 19 soit de 1400 grammes, celle du chlorure de potassium contenu dans le deuxième volume secondaire 20 soit de 67 grammes, celle du chlorure de magnésium contenu dans le troisième volume secondaire 21 soit de 47 grammes et celle du chlorure de calcium contenu dans le quatrième volume secondaire 22 soit de 73 grammes. De la sorte, il peut être produit de 5 à 6 litres de concentré liquide utilisé au cours d'une séance de dialyse.

A cette fin, la cartouche comporte une poche étanche 70 d'acide acétique 71, de 200 ml, diluée à 35 %, et qui est disposée dans le logement 50.

Dans ce dernier mode de réalisation il est alors avantageux que le couvercle 40 comporte au moins quatre orifices d'alimentation 44-47, ces quatre orifices étant respectivement disposés en regard des quatre volumes secondaires 19-22 définis ci-dessus.

Cependant dans le cadre de cette application à la dialyse, il est à remarquer que le premier volume secondaire 19, est d'une valeur relativement grande et comporte une quantité relativement importante de produit en poudre en l'occurrence 1400 grammes de chlorure de sodium.

Pour obtenir une dissolution plus rapide et plus homogène de ce produit par un fluide porteur comme de l'eau il est préférable que la cartouche selon l'invention, comporte, comme illustré sur les figures, cinq raccords fluidiques 23-26 et 28 montés en coopération avec la paroi de fond 6.

De ces cinq raccords, deux 23, 28 permettent de relier l'extérieur du récipient avec le premier volume secondaire 19, ces deux raccords 23 et 28 constituant respectivement une entrée du fluide de dissolution et l'autre la sortie de la solution une fois obtenue. En revanche, les trois autres raccords 20-22 permettant de mettre en communication l'extérieur 27 du récipient 1 avec respectivement les deuxième 20, troisième 21 et quatrième 22 volumes secondaires, constituent à la fois l'entrée du fluide de dissolution et la sortie de la solution une fois obtenue.

A la description faite ci-dessus, il est apparent qu'elle atteint les buts de la présente invention tels qu'ils ont été spécifiés au préambule de la présente description et d'autres qui ont été mentionnés le long de celle-ci.

Il est en outre précisé que l'utilisation d'une cartouche, notamment comme celle qui a été spécifiée dans son application à la dialyse ne sera pas spécifiquement décrite ici, car cette utilisation se déduit sans aucune difficulté de la description faite dans le document définissant de l'art antérieur et référencé au préambule des présentes en l'occurrence le US-A-5,318,750.

## Revendications

1. Cartouche permettant de réaliser une solution à usage médical par dissolution d'une pluralité de substances en poudre dans au moins un liquide, la cartouche comportant:
un récipient (1) définissant un volume principal (2), ledit récipient comportant une paroi latérale (3) de forme sensiblement de révolution délimitant, à une (4) de ses deux extrémités (4, 7), une ouverture (5), une paroi de fond (6) solidaire de l'autre extrémité (7) de ladite paroi latérale, les parois latérale et de fond délimitant ledit volume principal, un noyau central (8) de forme sensiblement de révolution, ledit noyau central étant solidaire, par une de ses deux extrémités (9, 10), de ladite paroi de fond (6) de façon que son axe de révolution (11) soit sensiblement confondu avec celui de la paroi latérale (3), la hauteur dudit noyau central étant sensiblement égale à celle de ladite paroi latérale pour définir un volume annulaire (12) dans ledit volume principal, au moins deux parois de séparation, solidaires de façon étanche de la paroi de fond (6), de la surface intérieure (17) de la paroi latérale (3) et de la surface extérieure (18) dudit noyau central (8) pour délimiter deux volumes secondaires dans ledit volume annulaire, et
au moins deux raccords fluidiques montés en coopération avec la paroi de fond, ces deux raccords fluidiques permettant de relier l'extérieur dudit récipient avec respectivement les deux volumes secondaires.

2. Cartouche selon la revendication 1, dans laquelle une couronne de protection (30), solidaire de ladite paroi de fond (6), entoure les deux dits raccords fluidiques, la hauteur de ladite couronne prise à partir de ladite paroi de fond étant supérieure à la hauteur desdits raccords fluidiques prise à partir de cette même paroi de fond.

3. Cartouche selon la revendication 1, dans laquelle en outre un couvercle (40) est apte à recouvrir l'ouverture (5) du récipient (1).

4. Cartouche selon la revendication 3, et qui comporte des moyens (41) pour indexer angulairement la position du couvercle (40) sur ladite ouverture (5) par rapport à ladite paroi latérale (3).

5. Cartouche selon la revendication 4, dans laquelle les moyens (41) pour indexer angulairement la position du couvercle (40) sur ladite ouverture (5) sont constitués par au moins une partie en saillie (42) réalisée sur l'un des deux éléments "couvercle" (40) et "paroi latérale" (3) et par une partie en creux (43) complémentaire de la partie en saillie, ladite partie en creux étant réalisée sur l'autre des deux éléments.

6. Cartouche selon la revendication 3, dans laquelle ledit couvercle (40) comporte au moins deux orifices d'alimentation, ces deux orifices étant respectivement disposés en regard des deux volumes secondaires.

7. Cartouche selon la revendication 1, dans laquelle en outre un logement (50) est réalisé dans ledit noyau central (8).

8. Cartouche selon la revendication 7, dans laquelle ledit logement (50) comporte une ouverture (51) réalisée au niveau de la paroi de fond (6).

9. Cartouche selon la revendication 1, et qui comporte :
quatre parois de séparation (13, 14, 15, 16) solidaires de façon étanche de la paroi de fond (6), de la surface intérieure (17) de la paroi latérale (3) et de la surface extérieure (18) dudit noyau central (8) pour délimiter quatre volumes secondaires (19, 20, 21, 22) dans ledit volume annulaire (12), et
au moins quatre raccords fluidiques (23, 24, 25, 26) montés en coopération avec la paroi de fond, ces quatre raccords fluidiques permettant de relier l'extérieur dudit récipient (1) avec respectivement les quatre volumes secondaires (19, 20, 21, 22).

10. Cartouche selon la revendication 9, dans laquelle les premier (19), deuxième (20)" troisième (21), et quatrième (22) volumes secondaires représentent respectivement 50%, 20%, 10% et 20% du volume annulaire (12).

11. Cartouche selon la revendication 10, dans laquelle le premier (19) volume secondaire ayant un volume représentant 50% du volume annulaire contient du chlorure de sodium, les deuxième (20) et quatrième (22) volumes secondaires ayant chacun un volume égal à 20% du volume annulaire contiennent respectivement du chlorure de potassium et du chlorure de calcium, et le troisième (21) volume secondaire, ayant un volume égal à 10% du volume annulaire contient du chlorure de magnésium.

12. Cartouche selon la revendication 11, dans laquelle les dimensions des premier (19), deuxième (20), troisième (21) et quatrième (22) volume secondaires sont respectivement déterminées pour que la masse du chlorure de sodium contenu dans le premier volume secondaire soit de 1400 grammes, celle du chlorure de potassium contenu dans le deuxième volume secondaire soit de 67 grammes, celle du chlorure de magnésium contenu dans le troisième volume secondaire soit de 47 grammes et celle du chlorure de calcium contenu dans le quatrième volume secondaire soit de 73 grammes.

13. Cartouche selon la revendication 9, dans laquelle en outre un logement (50) est réalisé dans ledit noyau central (8) et une poche d'acide acétique est disposée dans ledit logement

14. Cartouche selon la revendication 9, dans laquelle une couronne de protection (30), solidaire de la paroi de fond (6), entoure les quatre raccords fluidiques (23, 24, 25, 26), la hauteur de ladite couronne prise à partie de la paroi de fond étant supérieure à la hauteur des raccords fluidiques prise à partir de cette même paroi de fond.

15. Cartouche selon la revendication 9, dans laquelle en outre un couvercle (40) est apte à recouvrir l'ouverture (5) du récipient (1).

16. Cartouche selon la revendication 15, et qui comporte des moyens (41) pour indexer angulairement la position du couvercle (40) sur ladite ouverture (5) par rapport à ladite paroi latérale (3).

17. Cartouche selon la revendication 16, dans laquelle les moyens (41) pour indexer angulairement la position du couvercle (40) sur ladite ouverture (5) sont constitués par au moins une partie en saillie (42) réalisée sur l'un des deux éléments "couvercle" et "paroi latérale" et par une partie en creux (43) complémentaire de la partie en saillie, ladite partie en creux étant réalisée sur l'autre des deux éléments.

18. Cartouche selon la revendication 15, dans laquelle ledit couvercle (40) comporte au moins quatre orifices d'alimentation (44, 45, 46, 47), ces quatre orifices étant respectivement disposés en regard des quatre volumes secondaires (19, 20, 21, 22).

19. Cartouche selon la revendication 10, dans laquelle cinq raccords fluidiques (23, 24, 25, 26, 28) sont montés en coopération avec la paroi de fond (6), deux (23, 28) de ces cinq raccords permettant de relier l'extérieur du récipient avec le premier (19) volume secondaire (19), ces deux raccords constituant respectivement une entrée et une sortie de fluide, les trois autres raccords (24, 25, 26) permettant de mettre en communication l'extérieur (27) du récipient (1) avec respectivement les deuxième (20), troisième (21) et quatrième (22) volumes secondaires, chacun de ces trois raccords constituant à la fois une entrée et une sortie de fluide.

## Claims

1. Cartridge which allows a solution for medical use to be produced by dissolving a plurality of powder substances in at least one liquid, the cartridge comprising:
- a receptacle (1) which defines a principal volume (2), said receptacle comprising a lateral wall (3) which is substantially a form generated by revolution and delimits at one (4) of its two ends (4, 7) an opening (5), a base wall (6) which is integral with the other end (7) of said lateral wall, the lateral and base walls delimiting said principal volume, a central core (8) which is substantially a form generated by revolution, said central core being integral by one of its two ends (9, 10) with said base wall (6) such that its axis of revolution (11) is substantially identical to that of the lateral wall (3), the height of said central core being substantially equal to that of said lateral wall in order to define an annular volume (12) in said principal volume, at least two separation walls which are integral in a sealed manner with the base wall (6), with the interior surface (17) of the lateral wall (3) and with the exterior surface (18) of said central core (8) in order to delimit two secondary volumes in said annular volume, and
- at least two fluid connections which are mounted in cooperation with the base wall, these two fluid connections allowing the exterior of said receptacle to be connected to the two secondary volumes respectively.

2. Cartridge according to claim 1, in which a protection collar (30) which is integral with said base wall (6) surrounds the two said fluid connections, the height of said collar measured from said base wall being greater than the height of said fluid connections measured from this same base wall.

3. Cartridge according to claim 1, in which furthermore a cover (40) is able to cover the opening (5) of the receptacle (1).

4. Cartridge according to claim 3, and which comprises means (41) for angular indexation of the position of the cover (40) on said opening (5) relative to said lateral wall (3).

5. Cartridge according to claim 4, in which the means (41) for angular indexation of the position of the cover (40) on said opening (5) are formed by at least one projecting part (42) which is produced on one of the two "cover" (40) and "lateral wall" (3) elements and by a hollow part (43) which is complementary to the projecting part, said hollow part being produced on the other of the two elements.

6. Cartridge according to claim 3, in which said cover (40) comprises at least two supply holes, these two holes being disposed respectively opposite the two secondary volumes.

7. Cartridge according to claim 1, in which furthermore a housing (50) is produced in said central core (8).

8. Cartridge according to claim 7, in which said housing (50) comprises an opening (51) produced at the level of the base wall (6).

9. Cartridge according to claim 1, and which comprises:
- four separation walls (13, 14, 15, 16) which are integral in a sealed manner with the base wall (6), with the interior surface (17) of the lateral wall (3) and with the exterior surface (18) of said central core (8) in order to delimit four secondary volumes (19, 20, 21, 22) in said annular volume (12), and
- at least four fluid connections (23, 24, 25, 26) which are mounted in cooperation with the base wall, these four fluid connections allowing the exterior of said receptacle (1) to be connected to the four secondary volumes (19, 20, 21, 22) respectively.

10. Cartridge according to claim 9, in which the first (19), second (20), third (21) and fourth (22) secondary volumes represent 50%, 20%, 10% and 20% respectively of the annular volume (12).

11. Cartridge according to claim 10, in which the first (19) secondary volume having a volume representing 50% of the annular volume contains sodium chloride, the second (20) and fourth (22) secondary volumes each having a volume equal to 20% of the annular volume contain potassium chloride and calcium chloride respectively and the third (21) secondary volume having a volume equal to 10% of the annular volume contains magnesium chloride.

12. Cartridge according to claim 11, in which the dimensions of the first (19), second (20), third (21) and fourth (22) secondary volumes are determined respectively in order that the mass of the sodium chloride contained in the first secondary volume is 1400 grams, that of the potassium chloride contained in the second secondary volume is 67 grams, that of the magnesium chloride contained in the third secondary volume is 47 grams and that of the calcium chloride contained in the fourth secondary volume is 73 grams.

13. Cartridge according to claim 9, in which furthermore a housing (50) is produced in said central core (8) and a pocket of acetic acid is disposed in said housing.

14. Cartridge according to claim 9, in which a protection collar (30) which is integral with the base wall (6) surrounds the four fluid connections (23, 24, 25, 26), the height of said collar measured from the base wall being greater than the height of the fluid connections measured from this same base wall.

15. Cartridge according to claim 9, in which furthermore a cover (40) is able to cover the opening (5) of the receptacle (1).

16. Cartridge according to claim 15, and which comprises means (41) for angular indexation of the position of the cover (40) on said opening (5) relative to said lateral wall (3).

17. Cartridge according to claim 16, in which the means (41) for angular indexation of the position of the cover (40) on said opening (5) are formed by at least one projecting part (42) which is produced on one of the two "cover" and "lateral wall" elements and by a hollow part (43) which is complementary to the projecting part, said hollow part being produced on the other of the two elements.

18. Cartridge according to claim 15, in which said cover (40) comprises at least four supply holes (44, 45, 46, 47), these four holes being disposed respectively opposite the four secondary volumes (19, 20, 21, 22).

19. Cartridge according to claim 10, in which five fluid connections (23, 24, 25, 26, 28) are mounted in cooperation with the base wall (6), two (23, 28) of these five connections allowing the exterior of the receptacle to be connected to the first (19) secondary volume (19), these two connections forming respectively a fluid inlet and outlet, the three other connections (24, 25, 26) allowing the exterior (27) of the receptacle (1) to be placed in communication respectively with the second (20), third (21) and fourth (22) secondary volumes, each of these three connections forming at the same time a fluid inlet and outlet.

## Patentansprüche

1. Kartusche, zum Realisieren einer Lösung zur medizinischen Nutzung durch Auflösung einer Vielzahl von Substanzen in Pulverform in mindestens einer Flüssigkeit, wobei die Kartusche aufweist:
einen Behälter (1), der ein Hauptvolumen (2) festlegt, welcher aufweist, eine Seitenwand (3) von im Wesentlichen kreisförmiger Form, die an einem (4) ihrer beiden Enden (4, 7) eine Öffnung (5) begrenzt, eine Bodenwand (6), die mit dem anderen Ende (7) der Seitenwand verbunden ist, wobei die Seitenwand und die Bodenwand das Hauptvolumen begrenzen, einen zentralen Kern (8) von im Wesentlichen kreisförmiger Form, wobei der zentrale Kern durch eines seiner beiden Enden (9, 10) mit der Bodenwand (6) derart verbunden ist, dass seine Mittelachse (11) im Wesentlichen mit derjenigen der Seitenwand (3) verschmolzen ist, wobei die Höhe des zentralen Kerns im Wesentlichen gleich derjenigen der Seitenwand ist, um ein ringförmiges Volumen (12) im Hauptvolumen festzulegen, mindestens zwei Trennwände, die mit der Bodenwand (6), der Innenfläche (17) der Seitenwand (3) und der Außenfläche (18) des zentralen Kerns (8) verbunden sind, um zwei sekundäre Volumina in dem ringförmigen Volumen festzulegen, und
mindestens zwei fluidische Anschlüsse, die in Verbindung mit der Bodenwand angebracht sind, wobei diese beiden fluidischen Anschlüsse gestatten, das Äußere des Behälters mit den beiden sekundären Volumina jeweils zu verbinden.

2. Kartusche nach Anspruch 1,
bei der ein Schutzkranz (30), der mit der Bodenwand (6) verbunden ist, die beiden fluidischen Anschlüsse umgibt, wobei die Höhe des Kranzes, ausgehend von der Bodenwand, größer ist als die Höhe der fluidischen Anschlüsse ausgehend von derselben Bodenwand.

3. Kartusche nach Anspruch 1,
bei der außerdem ein Deckel (40) in der Lage ist, die Öffnung (5) des Behälters (1) zu bedecken.

4. Kartusche nach Anspruch 3,
die Mittel (41) aufweist, um die Position des Deckels (40) auf der Öffnung (5) winkelbezogen auf die Seitenwand (3) zu bestimmen.

5. Kartusche nach Anspruch 4,
bei der die Mittel (41) um die Position des Deckels (40) auf der Öffnung (5) winkelbezogen zu bestimmen, durch mindestens einen vorspringenden Abschnitt (42), der an dem einen der beiden "Deckel"- (40) und "Seitenwand" (3)-Elemente ausgeführt ist, und durch einen vertieften Abschnitt (43) ausgebildet sind, der mit dem vorspringenden Abschnitt komplementär ist, wobei der vertiefte Abschnitt an dem anderen der beiden Elemente ausgeführt ist.

6. Kartusche nach Anspruch 3,
bei der der Deckel (40) mindestens zwei Einfüllöffnungen aufweist, wobei diese beiden Öffnungen jeweils den beiden sekundären Volumina gegenüber platziert sind.

7. Kartusche nach Anspruch 1,
bei der außerdem eine Aufnahme (50) in dem zentralen Kern (8) ausgeführt ist.

8. Kartusche nach Anspruch 7,
bei der die Aufnahme (50) eine Öffnung (51) aufweist, die auf Höhe der Bodenwand (6) ausgeführt ist.

9. Kartusche nach Anspruch 1,
die aufweist:
vier Trennwände (13, 14, 15, 16), die mit der Bodenwand (6), der Innenfläche (17) der Seitenwand (3) und der Außenfläche (18) des zentralen Kerns (8) verbunden sind, um vier sekundäre Volumina (19, 20, 21, 22) in dem ringförmigen Volumen (12) abzugrenzen und
mindestens vier fluidische Anschlüsse (23, 24, 25, 26), die in Verbindung mit der Bodenwand angebracht sind, wobei diese vier fluidischen Anschlüsse gestatten, das Äußere des Behälters (1) mit den vier sekundären Volumina (19, 20, 21, 22) jeweils zu verbinden.

10. Kartusche nach Anspruch 9,
bei der das erste (19), zweite (20), dritte (21) und vierte (22) sekundäre Volumen entsprechend 50 %, 20 %, 10 % und 20 % des ringförmigen Volumens (12) repräsentieren.

11. Kartusche nach Anspruch 10,
bei der das erste sekundäre Volumen (19), das ein Volumen hat, das 50 % des ringförmigen Volumens darstellt, Natriumchlorid enthält, wobei das zweite (20) und vierte (22) sekundäre Volumen, die jeweils ein Volumen gleich 20 % des ringförmigen Volumens haben, jeweils Kaliumchlorid und Calciumchlorid enthalten, und das dritte (21) sekundäre Volumen, das ein Volumen gleich 10 % des ringförmigen Volumens hat, Magnesiumchlorid enthält.

12. Kartusche nach Anspruch 11,
bei der die Abmessungen des ersten (19), zweiten (20), dritten (21) und vierten (22) sekundären Volumens jeweils bestimmt sind, damit die Masse an Natriumchlorid, die in dem ersten sekundären Volumen enthalten ist, 1400 Gramm, die Masse an Kaliumchlorid, die in dem zweiten sekundären Volumen enthalten ist, 67 Gramm, die Masse an Magnesiumchlorid, die in dem dritten sekundären Volumen enthalten ist, 47 Gramm und die Masse an Calciumchlorid, die in dem vierten sekundären Volumen enthalten ist, 73 Gramm beträgt.

13. Kartusche nach Anspruch 9,
bei der außerdem eine Aufnahme (50) in dem zentralen Kern (8) ausgeführt ist und eine Essigsäurekammer in der Aufnahme angeordnet ist.

14. Kartusche nach Anspruch 9,
bei der ein Schutzkranz (30), der mit der Bodenwand (6) verbunden ist, die vier fluidischen Anschlüsse (23, 24, 25, 26) umgibt, wobei die Höhe des Kranzes ausgehend von der Bodenwand größer ist, als die Höhe der fluidischen Anschlüsse ausgehend von derselben Bodenwand.

15. Kartusche nach Anspruch 9,
in der außerdem ein Deckel (40) in der Lage ist, die Öffnung (5) des Behälters (1) zu bedecken.

16. Kartusche nach Anspruch 15,
die Mittel (41) aufweist, um die Position des Deckels (40) auf der Öffnung (5) winkelbezogen auf die Seitenwand (3) zu bestimmen.

17. Kartusche nach Anspruch 16,
bei der die Mittel (41), um die Position des Deckels (40) auf der Öffnung (5) winkelbezogen zu bestimmen, durch mindestens einen vorspringenden Abschnitt (42), der an einem der beiden "Deckel"- und "Seitenwand"-Elemente ausgeführt ist, und durch einen vertieften Abschnitt (43) ausgebildet sind, der mit dem vorspringenden Abschnitt komplementär ist, wobei der vertiefte Abschnitt an dem anderen der beiden Elemente ausgeführt ist.

18. Kartusche nach Anspruch 15,
bei der der Deckel (40) mindestens vier Einfüllöffnungen (44, 45, 46, 47) aufweist, wobei diese vier Öffnungen jeweils den vier sekundären Volumina (19, 20, 21, 22) gegenüber platziert sind.

19. Kartusche nach Anspruch 10,
bei der fünf fluidische Anschlüsse (23, 24, 25, 26, 28) in Verbindung mit der Bodenwand (6) angebracht sind, wobei zwei (23, 28) dieser fünf Anschlüsse gestatten, das Äußere des Behälters mit dem ersten (19) sekundären Volumen (19) zu verbinden, wobei diese beiden Anschlüsse jeweils einen Fluideingang und einen Fluidausgang bilden, wobei die drei anderen Anschlüsse (24, 25, 26) gestatten, das Äußere (27) des Behälters (1) mit jeweils dem zweiten (20) sekundären Volumen, dem dritten (21) sekundären Volumen und dem vierten (22) sekundären Volumen in Verbindung zu setzen, wobei ein jeder dieser drei Anschlüsse gleichzeitig einen Fluideingang und einen Fluidausgang bildet.
